# EUROPEAN PATENT SPECIFICATION

(11) **EP 2 558 562 B1**
(45) Date of publication and mention of the grant of the patent: **04.10.2017**
(21) Application number: 10714204.4
(22) Date of filing: 15.04.2010
(51) Int. Cl.: C12M 1/12, C12M 3/06

(54) **CELL CULTURE SYSTEM**
ZELLKULTURSYSTEM
SYSTÈME DE CULTURE CELLULAIRE

(43) Date of publication of application: 20.02.2013
(73) Proprietor: Fraunhofer-Gesellschaft zur Förderung der angewandten Forschung e.V., 80686 München (DE)
(72) Inventor: REIS, Christian, 67346 Speyer (DE); TRAUBE, Andreas, 72622 Nuertingen (DE); BRODE, Tobias, 73728 Esslingen (DE)
(74) Representative: Wohlfahrt, Jan Günther
(86) International application number: PCT/EP2010/002317
(87) International publication number: WO 2011/127945

(56) References cited:
- US-A- 5 578 490
- US-A- 5 652 142
- US-A1- 2002 189 374
- US-A1- 2008 194 017

## Description

The invention relates to a container for liquid or gel for being received in a carrier or vessel and in particular to a cell culture insert for being received in a microtiter plate to form a cell culture system suitable for the cultivation of adherent cells or three-dimensional tissues as well as uses thereof.

At present, artificial biological tissue constructs, for example, artificial skin or artificial cartilage replacement, are manufactured in merely very small batches, in most cases even only as individual products. Cells required for the manufacture of artificial biological tissue are generally obtained by biopsy. The cells are isolated and subject to a cell culture system for propagation. Currently, conventional cell culture systems comprise what is known as a multiwell or microtiter plate, in the surface of which a plurality of containers or depressions ('wells') are formed for parallel cultivation of several cell cultures in one carrier. The wells serve to receive beaker-shaped containers, which are known as cell culture inserts. In alternative setups there may be provided a single container to be received in a single well or vessel in a carrier, for example, for the preparation of a single larger cell or tissue sheath. Cell culture inserts are generally each provided with a bottom for the cells or tissue to rest and formed by a particular membrane for support and nutrition of the cells contained therein.

In currently used cell culture systems, there is the problem that cultivation of the cells requires a liquid medium covering the cells. This liquid medium is to be directly metered into the container and onto the cells. This can lead to damage, in particular to mechanically sensitive cells or tissues. When changing from a submerged cultivation to what is known as airlift cultivation, it is even necessary for the liquid medium covering the cell preparation to be mainly extracted by suction from the preparation. This may also lead to damage the cells or tissue and/or impair the quality of the end product obtained there from.

Further cell culture plates are disclosed in US 5,652,142 A and US 5,578,490 A.

The technical problem underlying the present invention is to provide means for easy and safe removal or addition of a liquid from or to a cell culture or biological tissue, for example a biopsate received in a container or insert that is suitable for both, manual and automated or partly automated cultivation of adherent cells and/or three-dimensional tissues including the addition or exchange of cell culture medium. More general, the technical problem is to exchange a liquid, in particular a cell culture medium, from a container in which a first liquid or gel, and in particular a cell culture or tissue, is received, without affecting the first liquid or gel while the liquid is removed from or filled in the inside of the container.

The technical problem is fully solved by a novel insert or container according to claim 1, preferably adapted for being inserted or received in a vessel or carrier or, more particular, in a well of a microtiter or multiwell plate for cell culture. The container, in particular the insert, according to the invention is adapted for retaining a first liquid or gel, in particular a cell culture or biological tissue, in its interior. The container or insert comprises at least one side wall element and a bottom wall element for enclosing the interior. The container or insert is preferably open for access at its upper end. The container or insert forms, for example, a cylindrical, cube or brick-shaped beaker-like vessel.

The container or insert according to the invention is primarily characterized in that either the side wall element or the bottom wall element or both wall elements comprise at least one or a plurality of capillary openings having a diameter of 100 µm to 1200 µm providing a selective fluid connection between the inside of the container or insert and its outside for at least one liquid or fluidic medium. The capillary openings according to the present invention are specifically adapted to selectively form a barrier for the exit of said first liquid or gel through the openings across the wall elements of the container, more particular in dependency of one or more physico-chemical conditions existing at the capillary opening. That is in other variants also in or in close vicinity to the capillary opening. The fluid connection may be established for the first liquid for a certain time period and/or under a certain condition or only after (not before) a certain condition is met.

Said physico-chemical condition may be further characterized in that it is selected from one or more of: specific chemical, biological and physical conditions and interactions between said first liquid and the capillary opening. In a more particular variant the physico-chemical condition is the wetting angle of the first liquid at (in or in close vicinity to) the capillary opening. The wetting angle is in particular primarily or solely dependent on one or more of the factors selected from: specific chemical, biological and physical interactions between the first liquid and the capillary opening.

In a more particular variant the physico-chemical condition is the hydrostatic or transmural pressure present at the level of the capillary opening.

In an embodiment the at least one capillary opening form a selective barrier for the first while, at the same time, the capillary openings are adapted to form a liquid conducting path across the wall elements for the second liquid, which is different from the first liquid or gel mainly in its physical and/or chemical properties that is in particular viscosity, wetting ability and/or wetting angle. In a particular embodiment the second liquid has a viscosity lower than that of the first liquid or gel.

The invention makes use of the physical properties of a capillary system which, without wishing to be bound to the theory, is used herein to allow selective passage for a liquid, dependent on its fluidic properties, in particular its viscosity and its wetting ability. In connection with the dimensions of the capillary opening, in particular the smallest cross section diameter of the opening, and in connection with the surface properties of the materials of the wall elements and the capillary with particular respect to wettability and in connection with the surface activity for the liquid, the capillary opening either allows or inhibits the passage of the liquid there through. It was surprisingly found, that such capillary openings in a container or insert serve as a selective permeation system which advantageously allow an easy access to the interior of the container for a particular group of liquid substances or compositions.

This novel selective permeation system allows selective or controlled transport of liquids by means of capillary action through capillary openings provided in the wall. The transport is primarily dependent on the viscosity of the liquid or gel employed. Viscosity index of the liquid is a major decisive factor for the selective passage. As such, the container according to the present invention is liquid-tight to liquids or gels with, for example, a comparingly high viscosity, but may be freely permeable for liquids with a lower viscosity.

In another particular variant the selection for passage is determined by the wetting ability of the liquid, in particular the physical or chemical interaction with the wall surface.

In a particular embodiment a liquid's passage across the wall can be controlled by raising or lowering the level of the liquid inside or outside the container. More particular, the capillary opening qualifies as an externally controllable valve or bleeder. In this particular embodiment the selectivity and the passage of the liquid through the capillary openings is further dependent on the transmural pressure, i.e. hydrostatic pressures on the inside and the outside of the wall element through which the capillary opening extends.

In a variant the invention provides for allowing a liquid enter or leave the inside of the container dependent on the hydrostatic pressure present at the (lowest) capillary opening, i.e. level of filling with respect to the level of the capillary opening. More particular, there is provided a novel method for filling or draining the container by simply raising the fill level above a certain level. According to this particular aspect of the invention, a liquid can be filled into the container, but is prevented from draining from the inside of the container. This is accomplished by keeping the fill level of the liquid inside the container low and thus keeping the hydrostatic pressure low to prevent a wetting of the capillary opening. By that, no capillary action is triggered and the container is kept fluid tight for that liquid. This is particularly effective, if the capillary openings of the container are dry, i.e. have not been fully wetted by a liquid. In the event the fluid level is raised to a higher fill level, either inside or outside of the container, the rising hydrostatic pressure may overcome the physico-chemical barrier at the capillary opening and eventually lead to a full wetting of the capillary thereby triggering capillary action to establish a fluid connection for that liquid across the wall element of the container. The invention foresees to control the passage of a liquid through the capillary opening by keeping the fill level of the liquid with respect to the capillary opening at a certain low level to prevent a fluid connection or by raising the fill level above to induce or install a fluid connection across the wall element of the container. A method for filling or draining the container or insert with a liquid, in particular a cell culture medium, can comprise the steps of: Provision of a container or insert according to the invention and filling the liquid into the container, thereby ensuring not to raise the fill level above a certain predetermined first level, such to prevent the liquid from fully wetting the one or more capillary openings of the container, more particular to prevent capillary action to establish a fluid connection across the wall element of the container. The first level is primarily determined by the physico-chemical conditions present, as outlined in more detail herein. To establish a preferable permanent fluid connection across the wall element for that liquid, the level of liquid is raised to a second level above said first level, such that full wetting of the capillary opening takes place and a fluid connection across the wall element is established.

The method allows easy draining of the liquid phase of a cell suspension dispensed into the container. By keeping the fill level of the suspension below said certain first level in the first place the container is liquid tight for that suspension. The cells in the suspension may be allowed to settle and eventually attach to the bottom, in particular to a culture membrane, of the container. The remaining overlaying liquid phase may then be drained from the container by raising the fill level above said certain level, either by dosing further liquid into the container or, more preferable, by raising the fill level on the outside of the container, such that, the preferably permanent fluid connection is established, thereby allowing the liquid phase of the suspension to drain from the container. This method advantageously avoids any direct mechanical load to the cells

The invention advantageously allows a novel and simple way of filling or emptying of a container or insert. The invention thus also pertains to a novel method for easy and safe exchange of liquids in an insert or container. More particular, the invention allows a highly viscous liquid or gel composition, in particular a biomatrix cell culture, biological tissue or the like to be retained within the container or insert, while a second liquid, in particular a liquid medium for cell culture, which preferably shall overlay the first liquid or gel retained within the container, to be repeatedly exchanged, applied or removed through the capillary openings very easily and with no disturbance of or harm to the first liquid or gel, in particular a biomatrix, a cell culture or biological tissue.

To improve the exchange of liquid between the interior of the insert and the well, a plurality of capillaries may be present. In a particular embodiment, the at least one capillary opening, and more particular all capillary openings, are comprised in the side wall elements of the container. In a particular variant, no other fluid connection across the wall of the container or insert is present.

The capillary opening, in particular its smallest cross section, is adapted to the physico-chemical properties of the liquid or gel, more particular its viscosity and/or wetting ability.According to the invention, the capillary opening may have a diameter of from 100 to 1,200 µm, more particular of from 200 to 1,000 µm, even more particular of from 600 to 800 µm. In a more specific variant thereof, the capillary opening has a diameter of about 600 µm.

In a particular embodiment the capillary opening is a bore in the wall element. In another particular embodiment the opening is a separate element provided in the wall. The capillary may be made of a particular material, for example glass, and/or a material which interacts with the liquid. The capillary may be arranged in the insert or embodied so as to be integrated with the insert.

To modify or improve the selectivity of the passage, the wall may further comprise a surface active coating or structure which is present in, at or in close vicinity to the capillary opening. Selectivity for a particular liquid may be achieved by chemically active groups or functions provided at, in or in close vicinity to the capillary opening. Such structures may interact chemically or physically or physicochemically with one or more compounds of the liquid or composition. Selectivity for a particular liquid may be increased, for example, by selective interaction with the surface modification. In a first variant the wetting angle is increased for a particular liquid. In an alternative variant the wetting angle is decreased for a particular liquid. In a particular variant the surface active coating or structure is a hydrophobic coating. In an alternative variant the surface active coating or structure is hydrophilic. Such coatings or structures are known as such and may comprise or consist of plasma-induced surface modifications or plasma-deposited coatings as well as nanoparticular surfaces and biologically active surface structures for cell-surface interaction, but are not limited thereto.

According to the invention the container or insert further comprises a membrane which is primarily located at or within the bottom wall element. A membrane according to the present invention is considered as "liquid-tight" as such and thus does not allow any liquid or gel to freely pass through the wall element of the container. The membrane allows nutrition and/or gases to be exchanged only by diffusion. In particularly serves the purpose of supporting a cell culture or biological tissue placed within the container or insert. Such membranes apt for cell culture are known in the art and typically comprise a pore diameter of less than 10 µm. In a more particular variant the pore diameter of a membrane according to the invention is 8 µm or less, more particular 5 µm or less and may be as small as 1 µm or 0.4 µm.

In the context of the present invention a "first liquid or gel", in a first embodiment, is a cell culture composition with or without cells. In a particular variant thereof, the first liquid or gel is a cell suspension, comprising biological cells in a suitable cell culture medium and primarily a high viscosity biomatrix composition. More particular, the biomatrix composition is a hydrogel, a collagen gel or the like. In another variant, the first liquid or gel is a, preferably solidified or jellified, cell-free biomatrix apt to receive cells to be seeded thereon or to receive a biological tissue or biopsate. Such a cell-free biomatrix may also be a hydrogel, collagen gel or the like. The first liquid or gel can be a single layered cell culture preferably to be seeded into the interior of the container as a suspension wherein the cells may be allowed to settle onto the container's bottom to form a single cell sheath. The first liquid or gel can be a three-dimensional multilayered tissue culture, comprising at least two layers of biological cells of different nature, which may be embedded in or seeded onto the above-identified cell-free biomatrix. The container can retain a biological tissue or biopsate which may be embedded in the above-identified liquid or gel culture medium or biomatrix.

It is to be understood that the first liquid or gel as well as the second liquid are not limited to particular components for culturing biological cells or tissues. The invention also encompasses further applications employing other types of liquids or gels, for example, chemical test reagents, samples or probes. In general, the present invention provides a container for receiving or retaining a first liquid or gel while allowing a second liquid with other physico-chemical properties, in particular lower viscosity, to freely flow in or out of the container. The container can provide for the retention of a cell suspension or for retaining single layered or a three-dimensional multilayered tissue or tissue culture. The container can also provide for retaining a gel matrix as a support for a cell culture or tissue. The second liquid may be a liquid cell culture medium to overlay the cell culture retained within the container for nutrition, which is to be easily exchanged or removed during cell cultivation. The container is useful for retention of a chemical test reagent or probe as the first liquid or gel composition. The second liquid, for which the wall elements of the container are permeable, may be a chemical probe or a sample which may react with the chemical test reagent. While the chemical test reagent is immobilized in the container, the probe or sample may freely flow in and out of the container through the capillary opening(s).

The invention further probe a novel method for cultivating cells in a cell culture system comprising at least one well, in particular a multiwell test plate. The method or process comprises at least the following steps or, in a more particular embodiment, consists thereof: In an initial step (a), a container or insert according to the invention is provided for receiving cells, a cell culture composition or a biological tissue or biopsate. In a further step (b) a first liquid or gel cell culture or cell culture composition, primarily comprising or consisting of a biomatrix, a cell suspension, and one or more of a three-dimensional tissue construct or a biopsate, is transferred into the container. The invention provides for the liquid or gel cell culture composition being prevented from exit through the wall elements of the container. In a further step (c), the first liquid or gel or tissue, respectively, is overlaid with a second liquid, in particular a liquid medium or medium composition to which the capillary openings of the container are adapted such that the second liquid is allowed to freely flow into or out of the container across the wall element through the capillary openings.

In a particular embodiment the level of the liquid media composition overlaying the liquid or gel inside the container or insert is determined or adjusted by the relative position of the at least one capillary opening along the side wall element of the container. The capillary opening serves as a level drain or spill over for the liquid medium; excess medium liquid with a level above the lowest arranged capillary opening may be allowed to exit the container, for example, into the well or vessel.

In a preferred embodiment a plurality of capillary openings is distributed along the side wall element to provide for a complete drainage of the second liquid overlaying the retained first liquid or gel. To achieve this, a capillary opening is provided at each particular level. More particular the openings are spaced apart from each other with respect to the level or height, i.e. the distance to the bottom element of the container, with the proviso that the spacing is equal to or less than the cross-section diameter of each capillary opening. This may be accomplished by providing capillary openings in one or more rows arranged in a skewed or thread or multithread like fashion along the side wall element, such that each level or height is represented by at least one capillary opening.

In a particular embodiment the liquid media composition enters the container and consecutively flows onto the liquid or gel culture composition from the outside, for example, from the well through the at least one capillary opening in the wall element. In a particular mode of operation, the container or insert may be dipped or received in a well or a comparable vessel, which is filled with the liquid medium such that the liquid enters the container through the capillary openings and in particular overlays the liquid or gel culture or culture composition resting within the container or insert. To force the liquid medium to enter or leave the container through the capillary opening, the level of medium may be raised for inducing a hydrostatic pressure gradient across the wall element (transmural pressure). The flow of liquid medium into or from the container may be controlled by the initial level of medium metered in the space between the container and the well. The inflow or outflow of medium comes to a stop as soon as the level inside the container is levelled off with the level in the well with respect to the position of the at least one capillary opening. Advantageously, this mode of operation does not require the use of any pipetting means for filling and/or replacing liquid media composition in a cell culture system. Advantageously, this mode avoids the metering of medium or corresponding liquids directly into the inside of the container or insert, thus preventing, for example, disturbance of harm to the cell culture retained therein. The liquid media composition may also be drained out of the container or insert through the capillary openings. This may be accomplished by lowering the level of the liquid in the well or vessel or by lifting the container or insert. By that, the liquid medium exits the container through the at least one capillary opening. Advantageously, this mode allows an easy and safe emptying or draining of liquid media or corresponding liquids from the container or insert, without disturbing the cell culture. The clearing or drainage of liquid from the inside of the container or insert and/or the filling of its inside with liquid through the at least one capillary opening in the wall element may be facilitated by raising the level of said liquid or medium above normal level in order to increase the hydrostatic pressure. Without wishing to be bound to the theory, the increase of transmural pressure, in particular hydrostatic pressure, supports "initiation" of the capillary effect, i.e. supports drawing of liquid into the capillary. Furthermore, the level or height of the capillary opening relative to the level of the liquid may initially determine the liquid's ability to pass through the capillary opening. The selectivity of the liquid passage through the capillary opening can be determined by the level of the liquid with respect to the position of the capillary opening within the wall element, in particular the side wall element.

In a variant thereof there is provided at least one additional opening in the wall elements which is a non-capillary opening, specifically designed to let pass all liquids and without the use of capillary effects dependent on physico-chemical properties of the liquids. This purge opening may be provided to the effect that in an initial phase the liquid medium may first enter the inside of the container or insert through that purge opening to fill the inside to a level above the level or position of the selective capillary opening. This provides for a hydrostatic pressure at the level of the capillary opening. By that, the capillary effect may be triggered. In a secondary phase of this initial step, the liquid that enters the inside via the purge opening may flow through the established selective fluid connection of the capillary opening whereby the level of the liquid is now levelled-off with the capillary opening, i.e. excess amounts of liquid entered through the purge opening may leave through that capillary opening. In this particular embodiment after an initial step liquid or medium exchange can easily be carried out through the established fluid connection.

According to the invention, a targeted exchange of liquid is available by suitably selecting the size or the flow cross section of the means for connecting the interior of the insert to the well in a targeted, liquid-conducting manner and selecting a suitable material for the insert as a function of the surface tension and the viscosity of the liquid to be conducted between the well and the interior of the insert. In this way, it is possible to design, for example, a three-dimensional tissue construct with a liquid which does not seep out of the interior of the insert, while a medium provided for the exchange of liquid between the interior of the insert and the well can pass through the connection unimpeded. A liquid which is supplied into the well and provided for the exchange of liquid between the interior of the insert and the well thus also infiltrates the interior of the insert. Liquid, which is required for example for carrying out submerged cultivation of cells received in the insert, therefore no longer has to be metered directly into the insert, i.e. immediately to the preparation received in the insert. Instead, the liquid can be added to the well via the metering opening.

Conversely, on account of the targeted, liquid-conducting connection between the interior of the insert and the well, the removal of a liquid medium from the well also results in the lowering of the level of liquid in the interior of the cell culture insert. Changing from submerged cultivation to airlift cultivation therefore no longer requires liquid to be suction-extracted directly from the interior of the insert and thus immediately from the cell preparation received in the insert. The risk of damage to the cell preparation can therefore be greatly reduced when the cell culture system according to the invention is used for the cultivation of cells by submerged cultivation or by a cultivation method requiring a change from submerged cultivation to airlift cultivation.

In a further aspect, the invention also provides a holder, adapted to be received in a carrier, in particular a multiwell test plate. The holder may comprise at least one container. Preferably, the holder carries a plurality of containers and is configured so as to allow the containers to be inserted into corresponding wells eventually formed in the carrier. In particular, the holder can be configured in such a way that the inserts carried by the holder are arranged at a distance from one another allowing the inserts to be inserted into corresponding wells formed in the carrier. The use of a holder carrying a plurality of inserts makes the inserts easier to handle; this is advantageous, in particular when the cell culture system according to the invention is used for the cultivation of cell preparations in relatively large batches. Furthermore, the holder can be provided with perforations arranged in suitable positions, thus allowing inserts which are originally interconnected subsequently to be separated from one another again. The holder can comprise a base plate or frame in which a plurality of receiving openings is formed for receiving the containers. Preferably, the arrangement of the receiving openings in the base plate corresponds to the arrangement of the wells present in a carrier, so that the carrier can be safely arranged in individual wells or vessels by simple insertion of the holder, with the inserts attached thereto. Regions of the base plate of the holder that are arranged between the containers may further serve as a cover for channels formed in the carrier for connecting a plurality of wells in a fluid-conducting manner. This allows the risk of contamination to be minimised. The inserts and the holder can be in the form of separate components. Preferably, however, the holder and the inserts are designed in one piece. A gripping means is provided on the holder for manual or automated handling of the holder and the containers carried by the holder. For example, the gripping means can be formed by a web extending substantially perpendicularly from a surface of the base plate of the holder that is remote from the carrier in the assembled state of a cell culture system. If desired or required, the holder can also have a plurality of gripping means. A gripping means attached to the holder makes the holder easier to handle and is therefore particularly advantageous if a cell culture system is used for the automated or partly automated cultivation of cell preparations in relatively large batches.

In a particular aspect the invention thus provides a multiwell plate based cell culture system, comprising a multiwell plate or a similar structure having a plurality of wells or vessels and a plurality of the containers or inserts according to the invention received in that wells for cultivating cells or tissue. A cell culture insert according to the invention may be embodied in a beaker-shaped manner and be provided in the region of a bottom with a liquid-tight membrane allowing the conveyance of nutrients and the like from a medium received in the well in the direction of a cell culture arranged on the membrane. The multiwell plate of the cell culture system according to the invention may also comprise a metering opening which is connected to the well in a fluid-conducting manner and in this way allows a liquid or medium to be supplied into the well and/or a liquid or medium to be removed from the well. For example, the metering opening can be formed in a multiwell plate and be connected to the well in a fluid-conducting manner via a suitably adapted channel. The liquid or medium may be added into the well or removed there from without this requiring the insert or container in the well to be moved from its position. As the metering opening in the cell culture system according to the invention may be formed in the carrier itself, its position is unambiguously defined and thus completely independent of the arrangement of the insert or container in the well. The cell culture system may be used without difficulty in an automated or partly automated cell cultivation method in which media are supplied or are removed there from automatically. In particular, damage, caused by non-unambiguously defined positioning of a container's metering opening, to an automatic or semiautomatic metering device and also cross-contamination caused by spraying can be reliably avoided. Just one or only a few metering openings connected to a plurality of wells in a fluid-conducting manner can be formed in the carrier or plate of the cell culture system. The carrier of the cell culture system may also be provided with a plurality of metering openings which are each individually connected to a well in a fluid-conducting manner. A first metering opening may serve as a media supply opening, for example, while a second metering opening may be provided for removing media from the well.

A plurality of wells may be formed in the carrier or plate. The cell culture system may be used in an advantageous manner for the manufacture of artificial tissue constructs in relatively large batches. In principle, each well formed in the carrier may be connected in a fluid-conducting manner to a separate metering opening for supplying a medium into the well and/or for removing a medium from the well. Preferably, however, a plurality of wells formed in the carrier are each connected to one another in a fluid-conducting manner. The fluid-conducting connection between the wells can be established via channels, for example, which can be formed in the carrier and extend between the wells. In such a configuration of the cell culture system according to the invention, merely a single metering opening for supplying a medium into the wells and/or for removing a medium from the wells has to be associated with the wells which are connected to one another in a fluid-conducting manner. It goes without saying that two metering openings, of which one is used, for example within an automated or partly automated cultivation method, for supplying media into the wells and the other is used for removing media from the wells, can also be associated with the wells which are connected to one another in a fluid-conducting manner.

Furthermore, a plurality of groups of wells may be formed in the carrier. The individual wells of a group are then preferably connected to one another in a fluid-conducting manner, for example via channels formed in the carrier, whereas there is no fluid-conducting connection between the groups. Such a configuration of the cell culture system is advantageous, in particular when the cell culture system is to be used for the cultivation of cell preparations in relatively large batches using various media or a margin separation within the carrier is desired. For example, a first group of wells connected to one another in a fluid-conducting manner can be supplied with a first medium via a first metering opening or first metering openings, whereas a second group of wells which are connected to one another in a fluid-conducting manner, but separated from the wells of the first group, can be supplied with a second medium via a second metering opening or second metering openings.

The container or insert and/or the holder is/are preferably made of a plastics material, such as for example PP, PET or PS. Preferably, these components are designed as injection-moulded components, allowing simple and economical production of the cell culture system. In a particularly preferred embodiment, the carrier, the insert and/or the holder of the cell culture system according to the invention is/are made of a cryopreservable material. This allows the tissue constructs or cells to be immediately deep-frozen.

The cell culture system can also comprise a cryopreservation vessel displaying high transfer of heat. The shape of the cryopreservation vessel is preferably adapted to the shape of the holder with the insert or inserts carried by the holder, so that the holder comprising the insert or inserts can easily be inserted into the cryopreservation vessel. This allows gentle and effective cryopreservation.

The cell culture system according to the invention is suitable, in particular, for use in the manufacture of artificial skin as an in-vitro test system or transplant or for the manufacture of artificial cartilage transplants. Furthermore, the cell culture system can also be used for the cultivation of cells to construct artificial skin or cartilage transplants. The system can be used for the cultivation of cells on a laboratory scale, but also for automated or partly automated cell cultivation.

The invention will now be described in greater detail with reference to the schematic drawings:
Figure 1 shows a schematic cross-sectional drawing of the carrier or insert according to the invention;
Figure 2 shows a plurality of containers or inserts for a cell culture system that are assembled in or by a holder;
Figure 3 shows a microwell plate as a carrier of a cell culture system with wells and received therein; and
Figure 4 shows an alternative embodiment of a single container to be received in a vessel or carrier.

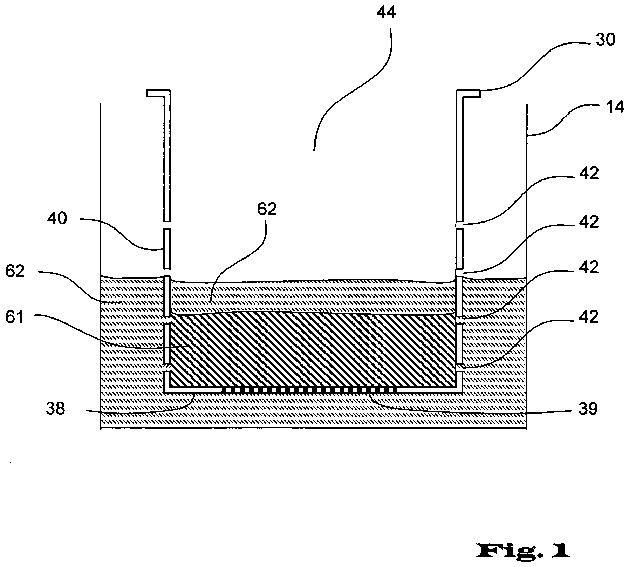

Figure 1 is a schematic cross sectional view of an insert or container (30) comprising capillary openings (42) according to the invention in at least one of the wall element (38, 40) of the container. The container (30) is designed to be received in a well (14) of a multiwell plate or in a similar vessel. The container (30) is primarily designed in a cylindrical or conical, rotation symmetric shape and comprises a side wall element (40) and a bottom wall element (38). The bottom wall element (38) may further comprise a preferably liquid-tight membrane (39) for allowing nutrients and gases to diffuse through the bottom part of the container (30). In a mode of operation, the container (30) retains a first liquid or gel (61) which is preferably a cell culture composition, for example, a cell culture matrix, cell suspension or a three-dimensional multilayered tissue construct. The first liquid or gel (61) is preferably overlaid by a second liquid (62), which, for example, resembles a cell culture medium for supporting growth and nutrition of cells embedded in or seeded on the first liquid or gel composition (61).

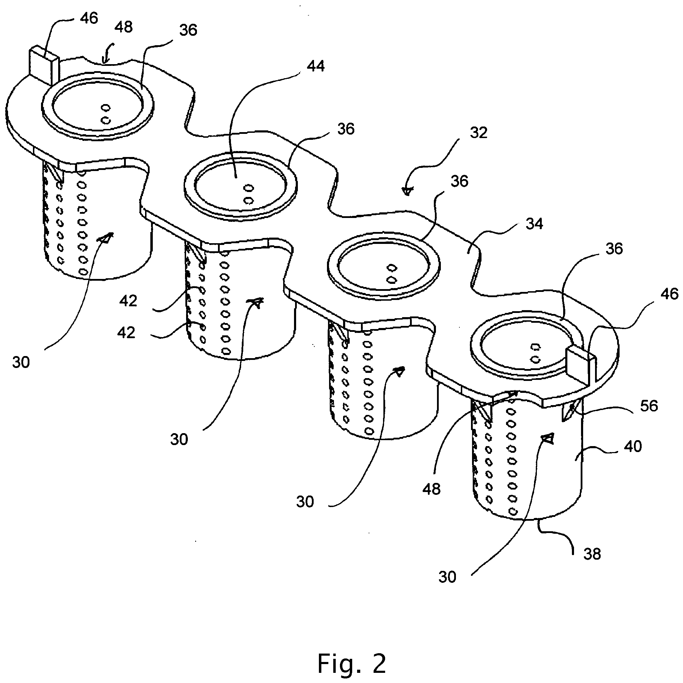

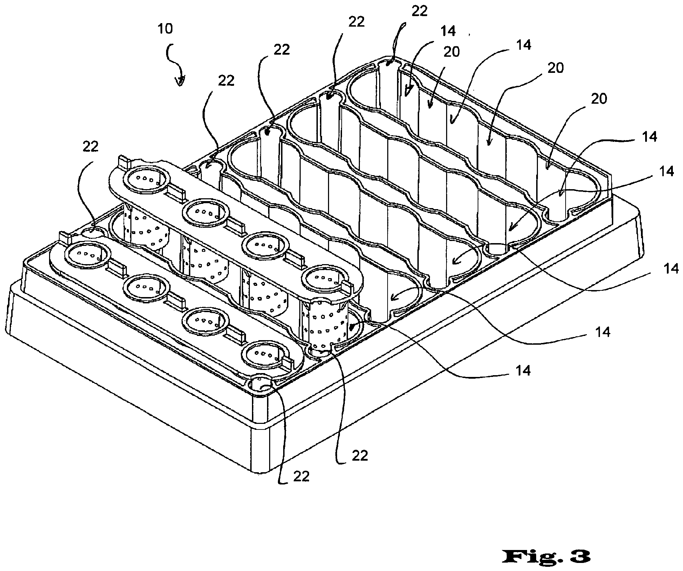

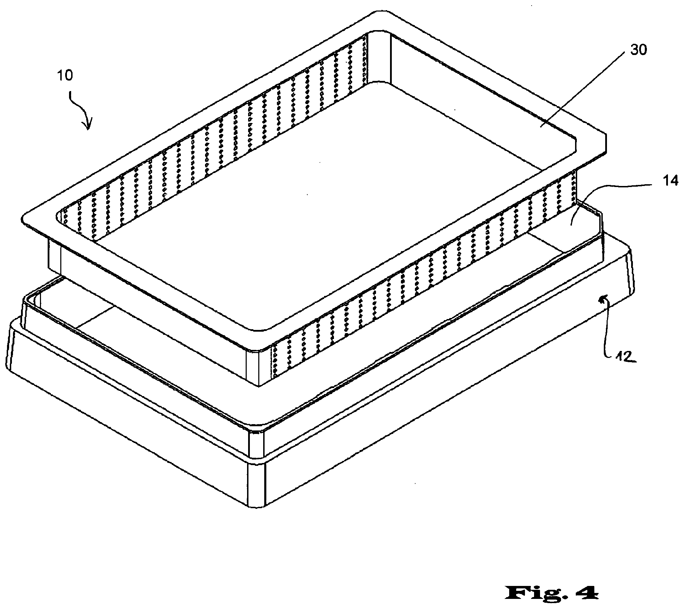

Figures 2 to 4 show oblique views of elements of cell culture systems, which are suitable in particular for the cultivation of adherent cells and/or three-dimensional tissues, is generally denoted by reference numeral (10). The cell culture system (10) comprises a carrier (12). According to the embodiment of Figure 3, six groups of wells (14) are formed in the carrier (12). Wells (14) of each group may each be connected to one another in a fluid-conducting manner by channels (20) extending between the wells (14). In the embodiment of Figure 3, there is no fluid-conducting connection between the individual groups of wells (14). It is thus possible to supply a different medium to the wells (14) of group I from that supplied to the wells (14).

Each insert (30) comprises a bottom (38) formed by a membrane for receiving a cell culture. A side wall (40) of the beaker-shaped inserts (30) is provided with a plurality of openings (42). When the inserts (30) are inserted, as shown in Figure 3 into the wells (14) formed in the carrier (12), the openings (42) formed in the side walls (40) of the inserts (30) allow liquid to be exchanged between the wells (14) and an interior (44) of the inserts (30).

A first metering opening (22) may be associated with each well (14) or each group of wells (14) for supplying media into the wells (14). The first metering opening (22) is formed in the carrier (12) and connected to a channel establishing a fluid-conducting connection between the first metering opening (22) and the wells (14).

The cell culture system (10) also comprises a plurality of inserts (30). Inserts (30) provided for insertion into a well (14) may be each formed in one piece with a holder (32). The holder (32) as depicted in Figures 2 and 3 comprises a base plate (34) in which one or a plurality of receiving openings (36) are formed for receiving inserts (30). The holder (32) and the at least one insert (30) may be formed in one piece and may be produced as an injection-moulded component.

The holder (32) carrying the at least one insert (30) may also be provided with one or a plurality of gripping means (46). The gripping means (46) are formed by webs which may extend substantially perpendicularly from a surface of the base plate (34) of the holder that is remote from the carrier (12) in the assembled state of the cell culture system (10) (see Figure 3). The gripping means (46) make the holder (32) or insert (30) easier to handle; this is advantageous, in particular when a cell culture system (10) is used in an automated or semi automated cultivation method.

In an assembled state of the cell culture system (10) as illustrated in Figure 3, the arrangement of the metering openings (22) in the carrier (12) allows media to be supplied into the wells (14) without this requiring the inserts (30) to be displaced from their position in the wells (14) or even to be removed from the wells (14). Furthermore, the position of the metering openings (22) is unambiguously defined and is not dependent on the position of the inserts (30) in the wells, so that the cell culture system (10) is well suited for use in an automated or partly automated cultivation method.

A selective liquid-conducting or fluid connection between the wells (14) and the interior (44) of the inserts 33 is established through the capillary openings (42) formed in the side wall (40) of each insert (30), the supplying of a medium into the wells (14) also allows media to be supplied into the interior (44) of the inserts (30). Similarly, a level of a liquid in the interior (44) of the inserts (30) can be lowered by removing media from the wells (14). The cell culture system (10) is therefore particularly well suited for the cultivation of cells by submerged cultivation or for use in a cultivation method requiring a change from submerged cultivation to airlift cultivation, as the exchange of liquid between the wells (14) and the interior (44) of the inserts (30) obviates the need to supply media directly into the interior (44) of the inserts (30) and also to remove media directly from the interior (44) of the inserts (30). Sensitive cell preparations can, in particular, be protected from damage in this way.

In the assembled state of the cell culture system (10), the base plate (34) of the holder (32) may cover the channels (20) extending between the wells (14), thus reliably preventing contamination of the medium received in the wells (14). However, when the inserts (30) carried by the holder (32) are inserted into the wells (14) formed in the carrier (12), recesses (48, 50) formed in the base plate (34) of the holder interact with the metering openings (22, 26) formed in the carrier plate (18), so that the base plate (34) of the holder does not obstruct unimpeded access to the metering openings (22, 26).

Ribs (56) may be connected to the side walls (40) of the inserts (30), which extend from the surface of the base plate (34) of the holder that faces the carrier (12) in the assembled state of the cell culture system (10). The ribs (46) may engage corresponding indentations or recesses in the carrier (12) or holder (32) to ensure proper and reproducible positioning to the insert (30) within the carrier (12) or holder (32). The ribs (56) may also stabilise the connection of the inserts (30) to the holder (32), so that the mechanical strength of the holder (32), with the inserts (30) fastened thereto, is advantageously increased.

The cell culture system (10) can also comprise a cryopreservation vessel (not shown in the drawings) displaying high transfer of heat. The shape of the cryopreservation vessel is adapted to the shape of the holder (32) with the inserts (30) which are embodied so as to be integrated therewith, so that the holder (32) comprising the inserts (30) can easily be inserted into the cryopreservation vessel. This allows gentle and effective cryopreservation.

## Claims

1. A container or insert (30), comprising a side wall element (40) and a bottom wall element (38) for enclosing an interior (44) for selectively retaining a first liquid or gel (61) in the interior (44) and adapted to be received in a well (14) of a carrier (12), **characterized in that** the side wall element comprises one or a plurality of capillary openings (42), specifically adapted to selectively form a barrier for the exit or entrance of the first liquid or gel (61) across the wall element (40) dependent on a physico-chemical condition existing at the capillary opening, wherein the capillary opening (42) has a diameter of 100 µm to 1200 µm and wherein the bottom wall element (38) comprises a liquid-tight membrane (39) for receiving the first liquid or gel (61) and for allowing nutrients and gases to diffuse through the bottom part of the container (30).

2. The container (30) according to claim 1, wherein the physicochemical condition is one or more of: specific chemical, biological and physical conditions and interactions between the first liquid and the capillary opening, preferably the physico-chemical condition is the wetting angle of the first liquid at the capillary opening, wherein the wetting angle is dependent on one or more of: specific chemical, biological and physical interactions between the first liquid and the capillary opening.

3. The container (30) according to one of the preceding claims, wherein the physico-chemical condition is the hydrostatic or transmural pressure.

4. The container (30) according to one of the preceding claims, wherein the capillary openings (42) form a liquid conducting opening across the wall element (40) for a second liquid (62) having at least one physico-chemical property different from that of the first liquid or gel (61), wherein the at least one physico-chemical property of the second liquid (62) is a lower viscosity and/or a greater wetting ability than that of the first liquid or gel (61).

5. The container (30) according to one of the preceding claims, wherein the capillary opening (42) has a diameter of 200 µm to 1000 µm.

6. The container (30) according to one of the preceding claims, wherein the wall element (40), comprises a surface active coating or structure in, at, or in vicinity to the capillary opening (42), wherein the surface active coating or structure is hydrophobic or the surface active coating or structure is hydrophilic.

7. The container (30) according to one of the preceding claims, wherein the first liquid or gel (61) is a chemical test reagent.

8. The container (30) according to one of the preceding claims, wherein the first liquid or gel (61) is a bio matrix for cell culture, a cell suspension, a multilayered tissue culture or a single layered cell sheath.

9. The container (30) according to one of the preceding claims, wherein the second liquid (62) is a chemical probe or sample or the second liquid (62) is a cell culture medium.

10. A holder (32), adapted to be received in a carrier (12), comprising a base frame (34) and one or a plurality of containers (30) according to any one of claims 1 to 9.

11. A cell culture system (10), comprising a carrier (12) having a plurality of wells (14) and the container (30) according to any one of claims 1 to 9 or the holder (32) according to claim 10 received in a well (14).

12. The cell culture system (10) of claim 11, wherein the carrier (12) further comprises one or more metering openings (22, 26) which are in fluid connection to the well (14) for supplying the second liquid (62) to the well (14).

13. Method for cultivating cells comprising the steps of:
- providing the container of any one of claims 1 to 9 for receiving cells,
- transferring a liquid or gel cell culture composition into the container whereby the liquid or gel composition is prevented from exit through the at least one capillary opening,
- overlaying the liquid or gel cell culture with a liquid medium which can freely flow into or out of the container via the at least one capillary opening.

14. Method according to claim 13 wherein the level of the liquid medium overlaying the liquid or gel cell culture composition in the container is determined by the relative position of the capillary openings in the side wall element of the container, wherein the liquid medium enters and/or exits the container through the at least one capillary opening.

15. Method according to one of claims 13 or 14, wherein the liquid medium is drained from the inside of the container and/or drawn into the inside of the container through the at least one capillary opening by initially raising the level of the liquid above normal level so as to increase the hydrostatic pressure and to neutralize capillary action and establish a fluid connection.

## Patentansprüche

1. Behälter oder Einsatz (30), umfassend ein Seitenwandelement (40) und ein Bodenwandelement (38) als Begrenzung eines Innenraums (44) für das selektive Zurückhalten einer ersten Flüssigkeit oder eines ersten Gels (61) in dem Innenraum (44) und angepasst, in einer Vertiefung (14) eines Trägers (12) aufgenommen zu werden, **dadurch gekennzeichnet, dass** das Seitenwandelement eine oder eine Mehrzahl von Kapillaröffnung(en) (42) umfasst, die speziell angepasst ist/sind zur selektiven Bildung einer Barriere für den Austritt oder Zutritt der ersten Flüssigkeit oder des ersten Gels (61) über das Wandelement (40) hinweg, in Abhängigkeit vom Vorliegen einer physikalisch-chemischen Bedingung an der Kapillaröffnung, wobei die Kapillaröffnung (42) einen Durchmesser von 100 µm bis 1200 µm besitzt und wobei das Bodenwandelement (38) eine flüssigkeitsdichte Membran (39) umfasst, die zur Aufnahme der ersten Flüssigkeit oder des ersten Gels (61) dient und die Diffusion von Nährstoffen und Gasen durch den Bodenteil des Behälters (30) ermöglicht.

2. Behälter (30) nach Anspruch 1, wobei es sich bei der physikalisch-chemischen Bedingung um eines oder mehr der folgenden handelt: spezielle chemische, biologische und physikalische Bedingungen und Wechselwirkungen zwischen der ersten Flüssigkeit und der Kapillaröffnung, wobei die physikalisch-chemische Bedingung vorzugsweise der Benetzungswinkel der ersten Flüssigkeit an der Kapillaröffnung ist, wobei der Benetzungswinkel von einem oder mehr der folgenden abhängig ist: spezielle chemische, biologische und physikalische Wechselwirkungen zwischen der ersten Flüssigkeit und der Kapillaröffnung.

3. Behälter (30) nach einem der vorherigen Ansprüche, wobei es sich bei der physikalisch-chemischen Bedingung um den hydrostatischen oder transmuralen Druck handelt.

4. Behälter (30) nach einem der vorherigen Ansprüche, wobei die Kapillaröffnungen (42) über das Wandelement (40) hinweg eine flüssigkeitsführende Öffnung für eine zweite Flüssigkeit (62) bilden, die mindestens eine physikalisch-chemische Eigenschaft besitzt, die sich von der der ersten Flüssigkeit oder des ersten Gels (61) unterscheidet, wobei es sich bei der mindestens einen physikalisch-chemischen Eigenschaft der zweiten Flüssigkeit (62) um eine niedrigere Viskosität und/oder eine größere Benetzbarkeit als bei der ersten Flüssigkeit oder dem ersten Gel (61) handelt.

5. Behälter (30) nach einem der vorherigen Ansprüche, wobei die Kapillaröffnung (42) einen Durchmesser von 200 µm bis 1000 µm besitzt.

6. Behälter (30) nach einem der vorherigen Ansprüche, wobei das Wandelement (40) eine oberflächenaktive Beschichtung oder Struktur in, an oder in der Nähe der Kapillaröffnung (42) umfasst, wobei die oberflächenaktive Beschichtung oder Struktur hydrophob ist oder die oberflächenaktive Beschichtung oder Struktur hydrophil ist.

7. Behälter (30) nach einem der vorherigen Ansprüche, wobei es sich bei der ersten Flüssigkeit oder dem ersten Gel (61) um ein chemisches Testreagenzien handelt.

8. Behälter (30) nach einem der vorherigen Ansprüche, wobei es sich bei der ersten Flüssigkeit oder dem ersten Gel (61) um eine Biomatrix für die Zellkultur, eine Zellsuspension, eine mehrschichtige Gewebekultur oder eine einschichtige Zellhülle handelt.

9. Behälter (30) nach einem der vorherigen Ansprüche, wobei es sich bei der zweiten Flüssigkeit (62) um eine chemische Sonde oder Probe handelt oder es sich bei der zweiten Flüssigkeit (62) um ein Zellkulturmedium handelt.

10. Halter (32), angepasst um in einem Träger (12) aufgenommen zu werden, umfassend einen Grundrahmen (34) und einen oder eine Mehrzahl von Behälter(n) (30) nach einem der Ansprüche 1 bis 9.

11. Zellkultursystem (10), umfassend einen Träger (12) mit einer Vielzahl von Vertiefungen (14) und den Behälter (30) nach einem der Ansprüche 1 bis 9 oder den Halter (32) nach Anspruch 10, der in einer Vertiefung (14) aufgenommen ist.

12. Zellkultursystem (10) nach Anspruch 11, wobei der Träger (12) darüber hinaus ein oder mehr Dosieröffnungen (22, 26) umfasst, die in Flüssigverbindung mit der Vertiefung (14) stehen, um die zweite Flüssigkeit (62) zu der Vertiefung (14) zu leiten.

13. Verfahren zum Kultivieren von Zellen umfassend die Schritte:
- Bereitstellen des Behälters nach einem der Ansprüche 1 bis 9 zur Aufnahme von Zellen,
- Überführung einer Flüssig- oder Gel-Zellkulturzusammensetzung in den Behälter, wobei ein Austreten der Flüssig- oder Gelzusammensetzung durch die mindestens eine Kapillaröffnung verhindert wird,
- Überdecken der Flüssig- oder Gel-Zellkultur mit einem flüssigen Medium, das durch die mindestens eine Kapillaröffnung ungehindert in den oder aus dem Container fließen kann.

14. Verfahren nach Anspruch 13, wobei der Pegel des flüssigen Mediums, das die Flüssig- oder Gel-Zellkulturzusammensetzung in dem Behälter überdeckt, bestimmt wird durch die relative Stellung der Kapillaröffnungen in dem Seitenwandelement des Behälters, wobei das flüssige Medium durch die mindestens eine Kapillaröffnung in den Behälter eintritt und/oder aus dem Behälter austritt.

15. Verfahren nach einem der Ansprüche 13 oder 14, wobei das flüssige Medium durch die mindestens eine Kapillaröffnung aus dem Innern des Behälters abgezogen wird und/oder ins Innere des Behälters gesogen wird, indem zunächst der Pegel der Flüssigkeit über den Normalpegel angehoben wird, um den hydrostatischen Druck zu erhöhen und den Kapillareffekt aufzuheben und eine Flüssigverbindung zu etablieren.

## Revendications

1. Récipient ou garniture (30), comprenant un élément de paroi latérale (40) et un élément de paroi inférieure (38) pour renfermer un intérieur (44) pour retenir sélectivement un premier liquide ou gel (61) dans l'intérieur (44) et adapté(e) à être reçu(e) dans un puits (14) d'un support (12), caractérisé(e) en ce que l'élément de paroi latérale comprend une ou une pluralité d'ouvertures capillaires (42), spécifiquement adaptées à former sélectivement une barrière pour la sortie ou l'entrée du premier liquide ou gel (61) en travers de l'élément de paroi (40) en fonction d'une condition physicochimique existant au niveau de l'ouverture capillaire, dans lequel/laquelle l'ouverture capillaire (42) a un diamètre de 100 µm à 1200 µm et dans lequel/laquelle l'élément de paroi inférieure (38) comprend une membrane étanche au liquide (39) pour recevoir le premier liquide ou gel (61) et pour permettre à des nutriments et gaz de se diffuser à travers la partie inférieure du récipient (30).

2. Récipient (30) selon la revendication 1, dans lequel la condition physicochimique est un ou plusieurs éléments parmi : des conditions et interactions chimiques, biologiques et physiques spécifiques entre le premier liquide et l'ouverture capillaire, de préférence la condition physicochimique est l'angle de mouillage du premier liquide au niveau de l'ouverture capillaire, dans lequel l'angle de mouillage est dépendant d'un ou plusieurs éléments parmi : des interactions chimiques, biologiques et physiques spécifiques entre le premier liquide et l'ouverture capillaire.

3. Récipient (30) selon une des revendications précédentes, dans lequel la condition physicochimique est la pression hydrostatique ou transmurale.

4. Récipient (30) selon une des revendications précédentes, dans lequel les ouvertures capillaires (42) forment une ouverture de conduction de liquide en travers de l'élément de paroi (40) pour un second liquide (62) ayant au moins une propriété physicochimique différente de celle du premier liquide ou gel (61), dans lequel l'au moins une propriété physicochimique du second liquide (62) est une viscosité inférieure et/ou une capacité de mouillage supérieure à celle du premier liquide ou gel (61).

5. Récipient (30) selon une des revendications précédentes, dans lequel l'ouverture capillaire (42) a un diamètre de 200 µm à 1000 µm.

6. Récipient (30) selon une des revendications précédentes, dans lequel l'élément de paroi (40) comprend un revêtement ou une structure tensioactif(ve) dans, au niveau ou à proximité de l'ouverture capillaire (42), dans lequel le revêtement ou la structure tensioactif(ve) est hydrophobe ou le revêtement ou la structure tensioactif(ve) est hydrophile.

7. Récipient (30) selon une des revendications précédentes, dans lequel le premier liquide ou gel (61) est un réactif de test chimique.

8. Récipient (30) selon une des revendications précédentes, dans lequel le premier liquide ou gel (61) est une matrice biologique pour culture cellulaire, une suspension cellulaire, une culture de tissu multicouches ou une gaine cellulaire monocouche.

9. Récipient (30) selon une des revendications précédentes, dans lequel le second liquide (62) est une sonde ou un échantillon chimique ou le second liquide (62) est un milieu de culture cellulaire.

10. Monture (32) adaptée à être reçue dans un support (12), comprenant un cadre de base (34) et un ou une pluralité de récipients (30) selon l'une quelconque des revendications 1 à 9.

11. Système de culture cellulaire (10) comprenant un support (12) ayant une pluralité de puits (14) et le récipient (30) selon l'une quelconque des revendications 1 à 9 ou la monture (32) selon la revendication 10 reçu(e) dans un puits (14).

12. Système de culture cellulaire (10) selon la revendication 11, dans lequel le support (12) comprend en outre une ou plusieurs ouvertures de comptage (22, 26) qui sont en connexion fluidique avec le puits (14) pour fournir le second liquide (62) au puits (14).

13. Procédé de mise en culture de cellules comprenant les étapes consistant à :
- fournir le récipient selon l'une quelconque des revendications 1 à 9 pour recevoir des cellules,
- transférer une composition de culture cellulaire liquide ou en gel dans le récipient moyennant quoi la composition liquide ou en gel est empêchée de sortir à travers l'au moins une ouverture capillaire,
- recouvrir la culture cellulaire liquide ou en gel d'un milieu liquide qui peut s'écouler librement dans le ou hors du récipient par le biais de l'au moins une ouverture capillaire.

14. Procédé selon la revendication 13, dans lequel le niveau du milieu liquide recouvrant la composition de culture cellulaire liquide ou en gel dans le récipient est déterminé par la position relative des ouvertures capillaires dans l'élément de paroi latérale du récipient, dans lequel le milieu liquide pénètre dans le et/ou sort du récipient à travers l'au moins une ouverture capillaire.

15. Procédé selon une des revendications 13 ou 14, dans lequel le milieu liquide est évacué de l'intérieur du récipient et/ou aspiré dans l'intérieur du récipient à travers l'au moins une ouverture capillaire en élevant initialement le niveau du liquide au-dessus du niveau normal de manière à augmenter la pression hydrostatique et à neutraliser l'action capillaire et établir une connexion fluidique.
